**Europäisches Patentamt**

**European Patent Office**

**Office européen des brevets**

(11) Veröffentlichungsnummer: **0 317 855 B1**

(12) **EUROPÄISCHE PATENTSCHRIFT**

(45) Veröffentlichungstag der Patentschrift: **18.12.91**

(51) Int. Cl.5: **A61K 31/54**, //(A61K31/54, 31:505)

(21) Anmeldenummer: **88118906.2**

(22) Anmeldetag: **12.11.88**

(54) Pharmazeutische Präparate.

(30) Priorität: **24.11.87 DE 3739779**

(43) Veröffentlichungstag der Anmeldung:
**31.05.89 Patentblatt 89/22**

(45) Bekanntmachung des Hinweises auf die
Patenterteilung:
**18.12.91 Patentblatt 91/51**

(84) Benannte Vertragsstaaten:
**AT BE CH DE FR GB IT LI NL SE**

(56) Entgegenhaltungen:
**EP-A- 0 205 865**
**US-A- 3 081 230**
**US-A- 3 449 496**
**US-A- 4 323 570**

(73) Patentinhaber: **Beiersdorf Aktiengesellschaft**
**Unnastrasse 48**
**W-2000 Hamburg 20(DE)**

(72) Erfinder: **Armah, Ben, Dr.**
**Hemmingstedter Weg 123 f**
**W-2000 Hamburg 52(DE)**
Erfinder: **Stenzel, Wolfgang, Dr.**
**Lerchenweg 8**
**W-2057 Reinbek(DE)**
Erfinder: **Plänitz, Vera, Dr.**
**Habichtstrasse 53**
**W-2359 Henstedt-Ulzburg(DE)**

**Beschreibung**

Gegenstand der Erfindung sind neue pharmazeutische Zusammensetzungen oder Präparate, ihre Verwendung und ihre Herstellung.

Die Verbindung 4-Chlor-6-methoxy-2-methyl-5-(2-imidazolin-2-yl)aminopyrimidin (Moxonidin) ist als zentral-wirkendes Antihypertonikum aus dem US-Patent 4 323 570 bekannt.

Die Verbindung 6-Chlor-3,4-dihydro-2H-1,2,4-benzothiadiazin-7-sulfonamid-1,1-dioxid ist als Hydrochlorothiazid bekannt und als Diuretikum eingeführt (Merck-Index, 10. Auflage, Seite 692).

Weiterhin ist die Verbindung 2,4,7-Triamino-6-phenylpteridin als Triamteren bekannt und ein anerkanntes Diuretikum, vergleiche US-A-3 081 230.

Hydrochlorothiazid und Triamteren werden in der Humanmedizin seit vielen Jahren aufgrund ihrer diuretischen Eigenschaften verwendet.

Obwohl Moxonidin sich als weitgehend frei von unerwünschten Nebenwirkungen erwiesen hat, erscheint eine Reduzierung der Dosis zur Verminderung dieser Nebenwirkungen sinnvoll, wenn die Wirkstärke erhalten bleibt.

Es wurde nun überraschend gefunden, daß eine neue pharmazeutische Zusammensetzung, die Moxonidin oder seine pharmakologisch unbedenklichen Salze und Hydrochlorothiazid enthält, außerordentlich günstige pharmakologische Eigenschaften aufweist.

Unter pharmakologisch unbedenklichen Säureadditionssalzen sind beispielsweise die Salze der d-Weinsäure, Maleinsäure, Fumarsäure, Bernsteinsäure, Milchsäure, Zitronensäure, Zimtsäure, Salicylsäure, Adipinsäure, Essigsäure, Propionsäure, p-Aminobenzoesäure, Methansulfonsäure, Schwefelsäure, Phosphorsäure, insbesondere das Hydrochlorid zu verstehen. Moxonidin wird vorzugsweise als Salz, insbesondere als Hydrochlorid eingesetzt.

Weiterhin wurde gefunden, daß eine neue pharmazeutische Zusammensetzung, die Moxonidin oder seine pharmakologisch unbedenklichen Salze, Hydrochlorothiazid und Triamteren enthält, unerwartete hervorragende Eigenschaften besitzt.

Die erfindungsgemäßen Zusammensetzungen oder Kombinationen haben eine über die Wirkung der Einzelkomponente hinausgehende, synergistische therapeutische Wirkung. Sie dienen insbesondere zur Behandlung der Hypertonie. Vorzugsweise sind die erfindungsgemäßen Kombinationen der Wirkstoffe Bestandteil einer Dosierungseinheit, wie beispielsweise Kapsel, Tablette, Filmtablette, Dragee, Suppositorium, Pille oder Ampulle, insbesondere aber einer Tablette oder Filmtablette, bevorzugt in den angegebenen Gewichtsverhältnissen.

Besonders bevorzugt wird eine pharmazeutische Zusammensetzung, die 1 Gewichtsteil Moxonidin und 30 bis 400, vorzugsweise 40 - 125, insbesondere 62,5 oder 125 Gewichtsteile Hydrochlorothiazid enthält.

Moxonidin ist ein zentral-wirkendes Antihypertonikum mit einer mittleren effektiven Dosis von 3 - 10 mg/kg p.o. Dieser blutdrucksenkende Effekt wurde nach oraler Applikation mittels plethysmographischer Methode an Ratten ermittelt.

Hydrochlorothiazid ist ein bewährtes Saluretikum mit schwacher, nicht immer ausreichender antihypertensiver Eigenschaft. Dosen von 1 - 100 mg/kg haben keine blutdrucksenkende Wirkung.

Es wurde nun überraschenderweise gefunden, daß die zusätzliche Gabe von Hydrochlorothiazid die blutdrucksenkende Wirkung von Moxonidin erheblich verstärkt. Wie aus dem nachfolgenden Versuchsbericht ersichtlich, wird eine Dosis von Moxonidin von 3 - 10 mg/kg auf 0,3 - 1 mg/kg herabgesetzt, wenn Hydrochlorothiazid gleichzeitig verabreicht wird. Der Wert dieser Kombination liegt darin, daß sie wirksamer ist als die Einzelkomponenten.

Die Verwendung der erfindungsgemäßen Zusammensetzung erlaubt daher eine wirksame antihypertensive Behandlung über einen längeren Zeitraum.

Bevorzugt enthalten die erfindungsgemäßen pharmazeutischen Zusammensetzungen 1 Gewichtsteil Moxonidin und 62,5 bis 125 Gewichtsteile Hydrochlorothiazid. Beispielsweise enthält eine erfindungsgemäße Dosierungseinheit wie eine Tablette oder ein Dragee 0,2 mg Moxonidin, 12,5 mg bis 25 mg, insbesondere 12,5 mg oder 25 mg Hydrochlorothiazid und übliche Hilfsstoffe.

Weiterhin wird eine Dosierungseinheit wie eine Tablette oder ein Dragee mit 0,1 mg Moxonidin, 6,25 mg bis 12,5 mg, insbesondere 6,25 oder 12,5 mg Hydrochlorothiazid und üblichen Hilfsstoffen bevorzugt.

Beim Erwachsenen verabreicht, kann die Tagesdosis der oral verabreichten Wirkstoffe beispielsweise 0,1 - 0,6 mg Moxonidin und entsprechende Mengen Hydrochlorothiazid, beispielsweise 12,5 bis 100 mg Hydrochlorothiazid betragen, vorzugsweise 0,2 mg Moxonidin und 12,5 mg Hydrochlorothiazid bis 0,4 mg Moxonidin und 25 mg Hydrochlorothiazid. Die vorstehend genannten Gewichtsverhältnisse und Mengen werden auch für die einzelne Dosierungseinheit bevorzugt.

Die obengenannten Dosen werden für die Behandlung der Hypertonie besonders bevorzugt.

Gegenstand der Erfindung ist auch eine pharmazeutische Zusammensetzung die Moxonidin, Hydrochlorothiazid und zusätzlich Triamteren enthält.

Bevorzugt wird eine solche, drei Wirkstoffe enthaltende Kombination, die 1 Gewichtsteil Moxonidin, 30 bis 400 Gewichtsteile Hydrochlorothiazid und 50 bis 800 Gewichtsteile Triamteren enthält.

Außerdem werden auch in dieser Kombination von drei Wirkstoffen die vorstehend genannten Mengen und Gewichtsverhältnisse für Moxonidin und Hydrochlorothiazid bevorzugt.

Besonders bevorzugt wird eine solche, drei Wirkstoffe enthaltende Kombination, die 1 Gewichtsteil Moxonidin, 45 bis 200 Gewichtsteile Hydrochlorothiazid und 90 bis 400 Gewichtsteile Triamteren, insbesondere aber 62,5 bis 125 Gewichtsteile Hydrochlorothiazid und 125 bis 250 Gewichtsteile Triamteren enthält.

Es wurde gefunden, daß die Kaliumausscheidung reduziert werden kann, wenn das in der Humanmedizin seit Jahren verwendete Saluretikum Triamteren der Kombination von Moxonidin und Hydrochlorothiazid zugesetzt wird.

Die Verwendung dieser drei Wirkstoffe enthaltenden erfindungsgemäßen Zusammensetzung ist daher in besonderem Maße, wie auch die zwei Wirkstoffe enthaltende Kombination, für eine wirksame antihypertensive Langzeitbehandlung geeignet.

Bevorzugt enthalten diese erfindungsgemäßen pharmazeutischen Zusammensetzungen 1 Gewichtsteil Moxonidin, 62,5 oder 125 Gewichtsteile Hydrochlorothiazid und 125 oder 250 Gewichtsteile Triamteren. Beispielsweise enthält eine erfindungsgemäße Dosierungseinheit wie eine Tablette oder ein Dragee 0,2 mg Moxonidin, 12,5 mg bis 25 mg, insbesondere 12,5 mg oder 25 mg Hydrochlorothiazid und 25 mg bis 50 mg Triamteren, insbesondere 25 mg oder 50 mg Triamteren sowie übliche Hilfsstoffe.

Weiterhin wird eine Dosierungseinheit wie eine Tablette oder ein Dragee mit 0,1 mg Moxonidin, 6,25 mg bis 12,5 mg, insbesondere 6,25 mg oder 12,5 mg Hydrochlorothiazid und 12,5 mg bis 25 mg, insbesondere 12,5 mg oder 25 mg Triamteren sowie üblichen Hilfsstoffen bevorzugt.

Beim Erwachsenen kann die Tagesdosis der oral verabreichten Wirkstoffe beispielsweise 0,1 - 0,6 mg Moxonidin und entsprechende Mengen Hydrochlorothiazid und Triamteren, beispielsweise 12,5 mg bis 100 mg Hydrochlorothiazid und 25 mg bis 200 mg Triamteren betragen, vorzugsweise 0,2 mg Moxonidin, 12,5 mg Hydrochlorothiazid und 25 mg Triamteren bis 0,4 mg Moxonidin, 25 mg Hydrochlorothiazid und 50 mg Triamteren. Die vorstehenden Gewichtsverhältnisse und Mengen werden auch für die einzelne Dosierungseinheit bevorzugt.

Die genannten Dosen beider Kombinationen werden bevorzugt für die Behandlung der genannten Krankheiten und werden für Hypertonie besonders bevorzugt:

Die erfindungsgemäßen Kombinationen der therapeutischen Wirkstoffe besitzen hohe pharmakologische Wirkung und sind wertvolle Arzneimittel, haben insbesondere eine diuretische und antihypertensive Wirkung und sind in der Humanmedizin insbesondere in der Therapie von Ödemen unterschiedlicher Genese, Adipositas, insbesondere ödematöse Adipositas, und zur Behandlung von refraktären Ödemen, Herzinsuffizienz, Linksherzhypertrophie, koronarer Herzkrankheit, insbesondere Angina pectoris und Arrhythmien, insbesondere katecholamin-induzierte Arrhythmien sowie in der Langzeitbehandlung der arteriellen Hypertonie geeignet.

Auch Verfahren zur Behandlung sowie Verfahren zur Herstellung von Mitteln zur Behandlung der vorstehend genannten Krankheiten umfaßt die Erfindung.

Die erfindungsgemäßen pharmazeutischen Zusammensetzungen können parenteral oder über den Verdauungstrakt verabreicht werden, wobei die orale Verabreichung bevorzugt ist.

Die vorliegende Erfindung betrifft ebenfalls pharmazeutische Zusammensetzungen oder Präparate, welche die erfindungsgemäßen Wirkstoffkombinationen oder pharmazeutisch verwendbare Salze der Wirkstoffe enthalten.

Die erfindungsgemäßen Wirkstoffkombinationen oder Zusammensetzungen können ohne weitere Zusätze verwendet werden, beispielsweise als Pulver oder abgefüllt in Kapseln. Vorzugsweise enthalten sie jedoch mindestens einen pharmazeutischen Hilfsstoff, vorzugsweise mindestens einen für die orale Verabreichung geeigneten **Hilfsstoff.**

Diese pharmazeutischen Zusammensetzungen oder Präparate können beispielsweise fest oder flüssig sein und in den bei der Humanmedizin üblicherweise verwendeten pharmazeutischen Formen vorliegen, beispielsweise als Tabletten, Dragees, Gelkügelchen, Granulat, trinkbare Suspension, Suppositorien, Pille, Sirup, Saft, Tropfen, Emulsion, Filmtablette, Puder, Pulver, Lösung und injizierbare Lösung, deren Herstellung nach den üblichen Verfahren erfolgen kann. Zu den Wirkstoffen können die bei diesen pharmazeutischen Zusammensetzungen üblicherweise verwendeten Hilfsstoffe beziehungsweise Excipientien wie Trägermaterialien und Bindemittel hinzugefügt werden, beispielsweise Talkum, kolloidales Siliciumdioxid, Gummi arabicum, Lactose, Stärke, Magnesiumstearate, Kakaobutter, wässrige oder nicht wässrige Vehikel, Fette tierischen oder pflanzlichen Ursprungs, paraffinische Derivate, Glykole, verschiedene Netz-, Dispergier- oder Emulgiermittel und/oder Konservierungsmittel.

Die Wirkstoffe können mit den Hilfsstoffen beziehungsweise Excipientien wie Trägermaterialien und Bindemitteln in üblicher Weise gemischt und naß oder trocken granuliert werden. Je nach Art der verwendeten Zusatzstoffe kann gegebenenfalls auch durch einfaches Mischen ein direkt tablettierbares Pulver erhalten werden. Das Granulat oder Pulver kann direkt in Kapseln abgefüllt oder vorzugsweise in üblicher Weise zu Tablettenkernen verpreßt werden.

Gegenstand der Erfindung sind auch die Herstellungsverfahren der Kombinationen beziehungsweise der Mittel, Präparate und Zusammensetzungen, die diese Kombinationen enthalten.

Die Verfahren zur Herstellung der pharmazeutischen Zusammensetzungen sind dadurch gekennzeichnet, daß man erfindungsgemäße Wirkstoffe oder Kombinationen gegebenenfalls zusammen mit einem pharmazeutischen Hilfsstoff zu einem pharmazeutischen Präparat verarbeitet.

Bevorzugt erfolgt die Herstellung von Tabletten in mehreren Verfahrensschritten:

a) Hydrochlorothiazid und gegebenenfalls Triamteren werden mit einem Trägermaterial, vorzugsweise Lactose, trocken gemischt.

b) Moxonidin oder ein Moxonidinsalz wird in einem Lösungsmittel oder Lösungsmittelgemisch (z.B. mit gleichen Volumenanteilen), vorzugsweise aus Wasser, Alkoholen und halogenierten Kohlenwasserstoffen (z.B. Methylenchlorid, Chloroform) bestehend, mit einem Bindemittel, vorzugsweise Polyvinylpyrrolidon (PVP), gegebenenfalls unter Wärmezufuhr gelöst.

c) Das gemäß Verfahrensschritt a) erhaltene Pulvergemisch wird mit der gemäß Verfahrensschritt b) erhaltenen Lösung befeuchtet. Die Masse wird geknetet und anschließend granuliert. Das Granulat wird vorzugsweise bei erhöhter Temperatur z.B. in einem Wirbelschichttrockner oder einem Hordenschrank getrocknet. Das getrocknete Granulat wird anschließend in einer Sieb- und Mahl-Maschine aufgebrochen.

d) Gegebenenfalls verwendete Hilfsstoffe wie Fließmittel, Füllstoffe, Gleitmittel, Trennmittel und Sprengmittel (FST-Komplex), werden vorzugsweise mit Stärken gemischt.

e) Das gemäß Verfahrensschritt c) erhaltene aufgebrochene Granulat wird mit der Mischung von d) zu einer preßfertigen Mischung gemischt und diese wird anschließend nach üblichen Verfahren zu Tabletten verpreßt.

Prozentangaben beziehen sich auf das Gewicht.

Die nachstehenden Beispiele erläutern die Erfindung ohne sie jedoch einzuschränken.

Pharmazeutische Zusammensetzungen

Beispiel 1

Man stellt Tabletten her, die der folgenden Formulierung entsprechen:

| Hydrochlorothiazid | 12,5 mg |
| Moxonidin | 0,2 mg |
| Excipient (q.s.) | 250 mg |
|---|---|
| (Excipient: Polyvinylpyrrolidon, Maisstärke, behandelte Stärke, Lactose, Magnesiumstearat, Talkum). | |

Beispiel 2

Man stellt Tabletten her, die der folgenden Formulierung entsprechen:

| Hydrochlorothiazid | 12,5 mg |
| Moxonidin | 0,1 mg |
| Excipient (q.s.) | 250 mg |
|---|---|
| (Excipient: Polyvinylpyrrolidon, Maisstärke, behandelte Stärke, Lactose, Magnesiumstearat, Talkum). | |

Beispiel 3

Man stellt Tabletten her, die der folgenden Formulierung entsprechen:

| Triamteren | 25 mg |
|---|---|
| Hydrochlorothiazid | 12,5 mg |
| Moxonidin | 0,2 mg |
| Excipient (q.s.) | 250 mg |
| (Excipient: Polyvinylpyrrolidon, Maisstärke, behandelte Stärke, Lactose, Magnesiumstearat, Talkum). | |

Beispiel 4

Man stellt Tabletten her, die der folgenden Formulierung entsprechen:

| Triamteren | 25 mg |
|---|---|
| Hydrochlorothiazid | 12,5 mg |
| Moxonidin | 0,1 mg |
| Excipient (q.s.) | 250 mg |
| (Excipient: Polyvinylpyrrolidon, Maisstärke, behandelte Stärke, Lactose, Magnesiumstearat, Talkum). | |

Beispiel 5

a) 6,25 kg Hydrochlorothiazid und 87,25 kg Lactose (oder 74,75kg Lactose und 12,5 kg Triamteren im Falle der Kombination von drei Wirkstoffen) werden trocken gemischt.

b) 0,1 kg Moxonidin in der Form des Hydrochlorids und 1 kg PVP werden in einem Lösungsmittelgemisch aus Wasser, Äthanol und Methylenchlorid unter Wärmezufuhr gelöst.

c) Das gemäß Verfahrensschritt a) erhaltene Pulvergemisch wird mit der gemäß Verfahrensschritt b) erhaltenen Lösung befeuchtet. Die Masse wird geknetet und anschließend granuliert. Das Granulat wird bei etwa 50 °C eine Stunde in einem Wirbelschichttrockner getrocknet. Das getrocknete Granulat wird anschließend in einer Sieb- und Mahl-Maschine aufgebrochen.

d) 0,4 kg einer Mischung aus üblichem Fließmittel, Sprengmittel und Trennmittel (FST-Komplex) wird mit 5 kg Stärke gemischt.

e) Das gemäß Verfahrensschritt c) erhaltene aufgebrochene Granulat wird mit der Mischung von d) zu einer preßfertigen Mischung gemischt und diese wird anschließend nach üblichen Verfahren zu Tabletten von 200 mg Gewicht verpreßt.

Analog werden Tabletten mit einem Gewicht von 100 mg und 300 mg erhalten.

Zusammensetzung von Tabletten mit einem Gewicht von 100 mg, 200 mg und 300 mg (in Gewichtsprozent):

| | a) | b) |
|---|---|---|
| Lactose | 87,25 | 74,75 |
| PVP | 1 | 1 |
| Stärke | 5 | 5 |
| FST-Komplex | 0,4 | 0,4 |
| Moxonidin | 0,1 | 0,1 |
| Hydrochlorothiazid | 6,25 | 6,25 |
| Triamteren | - | 12,5 |

Zur Behandlung der genannten Krankheiten, insbesondere der Hypertonie, können die beschriebenen Tabletten einmal, zweimal oder mehrmals täglich verabreicht werden.

Pharmakologische Untersuchungen

1. Senkung des arteriellen Blutdrucks unter verschiedenen Dosen von Moxonidin

Die Versuche wurden nach in der Literatur bekannten Verfahren durchgeführt (Armah, B.; Drug Research, Vol. 27, (II) 1977, page 1882-1884)

Tabelle 1

| Senkung des arteriellen Blutdrucks (in mm Hg) unter verschiedenen Dosen von Moxonidin | |
|---|---|
| Dosis (mg/kg p.o.) | Senkung des systolischen Druckes (mm Hg) |
| Moxonidin 0,3 | 10 |
| Moxonidin 1,0 | 20 |
| Moxonidin 3,0 | 50 |
| Moxonidin 10,0 | 60 |

2. Einfluß von Hydrochlorothiazid auf den systolischen Blutdruck wacher genetisch-hypertoner Ratten

Die Versuche wurden nach in der Literatur bekannten Verfahren durchgeführt (Armah, B.; Drug Research, Vol. 27, (II) 1977, page 1882-1884)

Tabelle 2

| Einfluß von Hydrochlorothiazid auf den systolischen Blutdruck wacher genetisch-hypertoner Ratten | |
|---|---|
| Dosis (mg/kg p.o.) | Senkung des systolischen Druckes (mm Hg) |
| 1 | ± 0 |
| 3 | ± 0 |
| 10 | ± 0 |
| 30 | 2 ± 4 |
| 60 | 4 ± 6 |

3. Einfluß der Kombination Moxonidin-Hydrochlorothiazid auf den systolischen Blutdruck wacher genetisch-hypertoner Ratten

Die Versuche wurden nach in der Literatur bekannten Verfahren durchgeführt (Armah, B.; Drug Research, Vol. 27, (II) 1977, page 1882-1884)

Tabelle 3

| Einfluß der Kombination Moxonidin-Hydrochlorothiazid auf den systolischen Blutdruck wacher genetisch-hypertoner Ratten | | | |
|---|---|---|---|
| Zusatz von Hydrochlorothiazid | Moxonidin-Dosen (Druck in mm Hg) | | |
| mg/kg | 0,3 mg/kg | 1 mg/kg | 3 mg/kg |
| 0 | 10 mm Hg | 20 mm Hg | 50 mm Hg |
| 30 | 30 mm Hg | 40 mm Hg | 50 mm Hg |
| 60 | 40 mm Hg | 50 mm Hg | über 50 mm Hg |

Aus den vorstehenden Tabellen folgt, daß die zusätzliche Gabe von Hydrochlorothiazid die blutdrucksenkende Wirkung von Moxonidin erheblich verstärkt, so daß sich bei der Behandlung der Hypertonie übliche Moxonidin-Dosierungen verringern lassen, wenn die Kombination verabreicht wird. Dadurch lassen sich die ohnehin schon geringen Nebenwirkungen beträchtlich herabsetzen. Es konnte nämlich gezeigt werden, daß eine Moxonidin-Dosis von 1 mg/kg keine sedierende Eigenschaft mehr besitzt.

Der Zusatz von Triamteren (30 mg/kg p.o.) zu der Kombination Moxonidin-Hydrochlorothiazid (3 + 30 mg/kg p.o.) führt zu einer zusätzlichen Senkung des systolischen Blutdrucks männlicher spontan-hypertensiver Ratten um mindestens 15 - 20%.

**Patentansprüche**

1. Pharmazeutische Zusammensetzung, enthaltend Moxonidin (4-Chlor-6-methoxy-2-methyl-5-(2-imidazolin-2-yl)aminopyrimidin) oder seine pharmazeutisch unbedenklichen Salze und Hydrochlorothiazid (6-Chlor-3,4-dihydro-2H-1,2,4-benzothiadiazin-7-sulfonamid-1,1-dioxid).

2. Pharmazeutische Zusammensetzung gemäß Anspruch 1, dadurch gekennzeichnet, daß sie zusätzlich Triamteren (2,4,7-Triamino-6-phenylpteridin) enthält.

3. Pharmazeutische Zusammensetzung gemäß Anspruch 1, dadurch gekennzeichnet, daß sie 1 Gewichtsteil Moxonidin und 30 bis 400 Gewichtsteile Hydrochlorothiazid enthält.

4. Pharmazeutische Zusammensetzung gemäß Anspruch 2, dadurch gekennzeichnet, daß sie 1 Gewichtsteil Moxonidin, 30 bis 400 Gewichtsteile Hydrochlorothiazid und 50 bis 800 Gewichtsteile Triamteren enthält.

5. Pharmazeutische Zusammensetzung gemäß Anspruch 1, dadurch gekennzeichnet, daß sie 1 Gewichtsteil Moxonidin und 62,5 bis 125 Gewichtsteile Hydrochlorothiazid enthält.

6. Pharmazeutische Zusammensetzung gemäß Anspruch 2, dadurch gekennzeichnet, daß sie 1 Gewichtsteil Moxonidin, 62,5 bis 125 Gewichtsteile Hydrochlorothiazid und 125 bis 250 Gewichtsteile Triamteren enthält.

7. Pharmazeutische Zusammensetzung nach einem der Ansprüche 1 bis 6, enthaltend für die orale Verabreichung geeignete pharmazeutische Hilfsstoffe.

8. Pharmazeutische Zusammensetzung nach einem der Ansprüche 1 bis 7 in Form von Tabletten oder Dragees, enthaltend 0,2 mg Moxonidin und 12,5 mg Hydrochlorothiazid.

9. Pharmazeutische Zusammensetzung nach einem der Ansprüche 1 bis 7 in Form von Tabletten oder Dragees, enthaltend 0,2 mg Moxonidin, 12,5 mg Hydrochlorothiazid und 25 mg Triamteren.

10. Pharmazeutische Zusammensetzung nach einem der Ansprüche 1 bis 9 zur diuretischen und/oder antihypertensiven Behandlung.

11. Verfahren zur Herstellung der pharmazeutischen Zusammensetzungen nach einem der Ansprüche 1 bis 10, dadurch gekennzeichnet, daß man Moxonidin und Hydrochlorothiazid gegebenenfalls zusammen mit einem pharmazeutischen Hilfsstoff zu einem pharmazeutischen Präparat verarbeitet.

12. Verfahren zur Herstellung der pharmazeutischen Zusammensetzungen nach einem der Ansprüche 1 bis 10, dadurch gekennzeichnet, daß man Moxonidin, Hydrochlorothiazid und Triamteren gegebenenfalls zusammen mit einem pharmazeutischen Hilfsstoff zu einem pharmazeutischen Präparat verarbeitet.

**Claims**

1. Pharmaceutical composition containing moxonidine (4-chloro-6-methoxy-2-methyl-5-(2-imidazolin-2-yl)-aminopyrimidine) or its pharmaceutically acceptable salts and hydrochlorothiazide (6-chloro-3,4-dihydro-2H-1,2,4-benzothiadiazine-7-sulphonamide 1,1-dioxide).

2. Pharmaceutical composition according to Claim 1, characterised in that it additionally contains triamterene (2,4,7-triamino-6-phenylpteridine).

3. Pharmaceutical composition according to Claim 1, characterised in that it contains 1 part by weight of moxonidine and 30 to 400 parts by weight of hydrochlorothiazide.

4. Pharmaceutical composition according to Claim 2, characterised in that it contains 1 part by weight of moxonidine, 30 to 400 parts by weight of hydrochlorothiazide and 50 to 800 parts by weight of triamterene.

5. Pharmaceutical composition according to Claim 1, characterised in that it contains 1 part by weight of moxonidine and 62.5 to 125 parts by weight of hydrochlorotriazide.

6. Pharmaceutical composition according to Claim 2, characterised in that it contains 1 part by weight of moxonidine, 62.5 to 125 parts by weight of hydrochlorothiazide and 125 to 250 parts by weight of triamterene.

7. Pharmaceutical composition according to one of Claims 1 to 6, containing pharmaceutical inactive ingredients suitable for oral administration.

8. Pharmaceutical composition according to one of Claims 1 to 7 in the form of tablets or sugar-coated tablets containing 0.2 mg of moxonidine and 12.5 mg of hydrochlorothiazide.

9. Pharmaceutical composition according to one of Claims 1 to 7 in the form of tablets or sugar-coated tablets containing 0.2 mg of moxonidine, 12.5 mg of hydrochlorothiazide and 25 mg of triamterene.

10. Pharmaceutical composition according to one of Claims 1 to 9 for diuretic and/or antihypertensive treatment.

11. Process for the preparation of the pharmaceutical compositions according to one of Claims 1 to 10, characterised in that moxonidine and hydrochlorothiazide are processed, where appropriate together with a pharmaceutical inactive ingredient, to a pharmaceutical product.

12. Process for the preparation of the pharmaceutical compositions according to one of Claims 1 to 10, characterised in that moxonidine, hydrochlorothiazide and triamterene are processed, where appropriate together with a pharmaceutical inactive ingredient, to a pharmaceutical product.

## Revendications

1. Composition pharmaceutique contenant de la moxonidine (4-chloro-6-méthoxy-2-méthyl-5-(2-imidazolin-2-yl)aminopyrimidine) ou ses sels pharmaceutiquement acceptables et de l'hydrochlorothiazide (1,1-dioxyde de 6-chloro-3,4-dihydro-2H-1,2,4-benzothiadiazine-7-sulfonamide).

2. Composition pharmaceutique suivant la revendication 1, caractérisée en ce qu'elle contient, en outre, du triamtérène (2,4,7-triamino-6-phénylptéridine).

3. Composition pharmaceutique suivant la revendication 1, caractérisée en ce qu'elle contient 1 partie en poids de moxonidine et 30 à 400 parties en poids d'hydrochlorothiazide.

4. Composition pharmaceutique suivant la revendication 2, caractérisée en ce qu'elle contient 1 partie en poids de moxonidine, 30 à 400 parties en poids d'hydrochlorothiazide et 50 à 800 parties en poids de triamtérène.

5. Composition pharmaceutique suivant la revendication 1, caractérisée en ce qu'elle contient 1 partie en poids de moxonidine et 62,5 à 125 parties en poids d'hydrochlorothiazide.

6. Composition pharmaceutique suivant la revendication 2, caractérisée en ce qu'elle contient 1 partie en poids de moxonidine, 62,5 à 125 parties en poids d'hydrochlorothiazide et 125 à 250 parties en poids de triamtérène.

7. Composition pharmaceutique suivant l'une quelconque des revendications 1 à 6, contenant des adjuvants pharmaceutiques propres à l'administration par voie orale.

8. Composition pharmaceutique suivant l'une quelconque des revendications 1 à 7, sous la forme de comprimés ou de dragées contenant 0,2 mg de moxonidine et 12,5 mg d'hydrochlorothiazide.

9.  Composition pharmaceutique suivant l'une quelconque des revendications 1 à 7, sous la forme de comprimés ou de dragées contenant 0,2 mg de moxonidine, 12,5 mg d'hydrochlorothiazide et 25 mg de triamtérène.

10. Composition pharmaceutique suivant l'une quelconque des revendications 1 à 9, pour le traitement diurétique et/ou antihypertenseur.

11. Procédé de fabrication des compositions pharmaceutiques suivant l'une quelconque des revendications 1 à 10, caractérisé en ce qu'on convertit de la moxonidine et de l'hydrochlorothiazide, le cas échéant conjointement avec un adjuvant pharmaceutique, en un produit pharmaceutique.

12. Procédé de fabrication des compositions pharmaceutiques suivant l'une quelconque des revendications 1 à 10, caractérisé en ce qu'on convertit de la moxonidine, de l'hydrochlorothiazide et du triamtérène, le cas échéant conjointement avec un adjuvant pharmaceutique, en un produit pharmaceutique.